# EUROPEAN PATENT APPLICATION

(11) **EP 2 647 383 A1**
(43) Date of publication of application: **09.10.2013**
(21) Application number: 11844849.7
(22) Date of filing: 01.12.2011
(51) Int. Cl.: A61K 33/42, A61K 9/16, A61P 17/00, A61P 43/00

(54) **BREAST ENHANCEMENT ACCELERATOR**

(30) Priority: 01.12.2010 JP 2010268837
(71) Applicant: SofSera Corporation, Tokyo 160-0022 (JP)
(72) Inventor: KAWABE, Karl Kazushige, Tokyo 160-0022 (JP); KOGAI, Yasumichi, Tokyo 160-0022 (JP); ISHIKAWA, Yumiko, Tokyo 160-0022 (JP); KOMAMI, Aya, Tokyo 160-0022 (JP)
(74) Representative: HOFFMANN EITLE
(86) International application number: PCT/JP2011/077743
(87) International publication number: WO 2012/074037

(57) **Abstract**

A novel breast enlargement promoter is provided.

The present inventor perfected the present invention having discovered a novel attribute in which collagen production is promoted when fine particles of calcium phosphate come into contact with fibroblasts, and having further discovered a novel attribute in which breast enlargement effect is obtained by application of such fine particles to the breasts. More specifically, the present invention is a breast enlargement promoter characterized by containing fine particles of calcium phosphate.

## Description

### Technical Field

The present invention relates to a novel breast enlargement promoter.

### Background Art

A desire of a woman to obtain beautiful breasts or big breasts is universal. To fulfill the desire, researches for a breast enlargement method have been done. Examples of widely known breast enlargement methods include, for example, a method in which fat is injected into the breast, a method in which hyaluronic acid is injected, a method in which a bag filled with physiological saline, silicon, etc. is put into the breast.

In addition to those described above, researches to obtain breast enlargement effects by a skin-topical composition are performed. For example, Patent Literature 1 discloses a skin-topical composition for breast enlargement, which contains one kind, or two or more kinds of plant extracts extracted from rehmannia root, Japanese pepper, Chinese mushroom, salvia, pueraria root, Asiasarum root, peach kernel, and Cinnamon and cassia. The fat synthesis is purportedly promoted by using the composition, whereby to obtain breast enlargement effects.

In addition, Patent Literature 2 discloses a composition for breast enlargement that contains collagen as an active ingredient. The composition is purportedly usable as an external agent or supplement, and gives resilience to the breast, allowing to obtain a beautiful breast and excellent breast enlargement effects such as improvement of the cup size. Furthermore, the collagen purportedly makes it possible to enhance water retention and elasticity of the skin, and obtain beautiful skin effects such giving moisture to the skin at the same time.

### Citation List

### Patent Literature

[Patent Literature 1] Japanese Patent Application Laid-Open (JP-A) No. 2000-302667
[Patent Literature 2] JP-A No. 2008-044890

### Summary of Invention

### Technical Problem

The object of the present invention is to provide a novel breast enlargement promoter.

### Means for solving the problem

The present inventors have found a novel attribute of a fine particle of calcium phosphate to promote collagen production by contact with a fibroblastic cell, and further found a novel attribute of the fine particle to give breast enlargement effects by being applied to the breast, and completed the present invention.

The invention (1) is a breast enlargement promoter, which contains fine particles of calcium phosphate.

The invention (2) is the breast enlargement promoter of the invention (1), wherein the fine particle of calcium phosphate is a fine particle of hydroxyapatite.

The invention (3) is the breast enlargement promoter of the inventions (1) or (2), wherein the average particle size of the fine particle of calcium phosphate is 10 to 1,000 nm.

The invention (4) is the breast enlargement promoter of any one of the inventions (1) to (3), wherein the fine particle of calcium phosphate is a sintered body.

The invention (5) is the breast enlargement promoter of the invention (4), wherein the sintered body is manufactured by a method including
a mixing step in which a primary particle containing calcium phosphate is mixed with a fusion inhibitor, and
a sintering step in which the mixed particle obtained by the mixing step defined above is exposed to a sintering temperature.

The invention (6) is the breast enlargement promoter of any one of the inventions (1) to (5), wherein the breast enlargement promoter is for external use.

### Advantageous effect of the invention

The present invention takes effects of promoting collagen production by acting on a fibroblastic cell, and thus takes effects of improving the tension of the breast epidermis and the resilience of the breast by being applied to the breast to give effects of promoting breast enlargement. Particularly, the present invention is effective with respect to the breast hung down with aging and the like, and takes effects of improving the hanging down.

According to the invention (2), the invention (2) takes effects of particularly promoting collagen production by acting on a fibroblastic cell, and effects of promoting breast enlargement.

According to the invention (3), the average particle size of the fine particle of calcium phosphate is very small, and thus the invention (3) takes effects such as an effect of promoting breast enlargement by being externally applied to the breast.

According to the invention (4), action of increasing noticeable amount of collagen production is recognized by making the fine particle of calcium phosphate a sintered body, and thus the invention (4) takes effects such as a particularly high effect of promoting breast enlargement.

According to the invention (5), the fine particle of calcium phosphate are hardly fused with each other despite the sintering and the state of the primary particle is kept, to give a sintered body with the particle size being small, and thus the invention (5) takes effects of exerting further higher effects of promoting breast enlargement.

### Brief Description of Drawings

Fig. 1 is a graph that shows the results of the test using the breast enlargement promoter according to the present Example.
Fig. 2 is an echo cross-sectional photograph after application of the breast enlargement promoter according to the present Example to subject A over 2 months.
Fig. 3 is an echo cross-sectional photograph after application of the breast enlargement promoter according to the present Example to subject B over 2 months.

### Description of Embodiments

The breast enlargement promoter according to the present best embodiment contains a fine particle of calcium phosphate. In addition to this, the breast enlargement promoter suitably contains other breast enlargement-promoting active ingredients, alcohols, sugars, proteins, amino acids, water-soluble vitamins, fat-soluble vitamins, lipid, mucopolysaccharides or a surfactant, as an optional component.

Examples of the fine particles of the calcium phosphate according to the present best embodiment include, for example, hydroxyapatite (Ca₁₀(PO₄)₆(OH)₂), fluoroapatite (Ca₁₀(PO₄)₆F₂), and Ca₁₀(PO₄)₆Cl₂ and so on. In addition, the calcium phosphate may contain a compound in which a portion of the calcium ion and/or the hydroxy ion and/or the phosphate ion is substituted with strontium ion, barium ion, sodium ion, bicarbonate ion, carbonate ion, fluoride ion, chloride ion and the like, or tricalcium phosphate (Ca₃(PO₄)₂), calcium metaphosphate (Ca(PO₃)₂) or calcium octaphosphate (OCP). Among the examples described above, hydroxyapatite is suitable. And, Ca₁₀(PO₄)₆ (OH)₂ is suitably present on the surface of the calcium phosphate particle (particle of calcium phosphate) according to the present best embodiment. This Ca₁₀(PO₄)₆(OH)₂ may be suitably present on the surface of the calcium phosphate, and may be contained in 0.1% by weight or so, more preferably 50% by weight or more with respect to the total amount of calcium phosphate. In addition, the fine particle of calcium phosphate described above may contain tricalcium phosphate and the like, which are generated when sintering amorphous hydroxyapatite as described below. The calcium phosphate according to the present embodiment is excellent in affinity with the living tissue and stability in the biological environment.

The average particle size of the fine particle of calcium phosphate according to the present best embodiment is suitably 10 to 1,000nm, more suitably 20 to 300 nm, and even more suitably 20 to 250 nm. Such range of the particle size allows the fine particle of calcium phosphate to permeate the dermic layer from the surface of the skin and thus act on the fibroblastic cell contained in the dermic phase even as a topical agent of the breast enlargement promoter, and exert collagen promotion action, whereby to further exert effects of promoting breast enlargement. The range of 20 to 250 nm particularly allows the fine particle of calcium phosphate to take effects of easily permeating the dermic layer from the surface of the skin since the gap of the epidermic cell is around 250 nm. The variation coefficient is suitably 20% or less, more suitably 18% or less, and even more suitably 15% or less. Meanwhile, the average particle size and the variation coefficient may be calculated by measuring the particle sizes of at least 100 or more of the primary particles using an electronic microscope. Meanwhile, the "variation coefficient" is a value that shows the variation of the particle sizes of the particles, which can be calculated with standard deviation ÷ average particle size × 100 (%). The shape of the fine particle of calcium phosphate is not particularly limited, and may be, for example, a particle shape or a rod shape. Meanwhile, when the shape of the fine particle of calcium phosphate is the rod shape, the average particle size is measured by the major axis of the particle.

The fine particles of the calcium phosphate according to the present best embodiment is suitably a sintered body of calcium phosphate by sintering (firing) calcium phosphate (also called calcium phosphate ceramics). Particularly, the sintering is suitably performed by the dispersion firing method described below. Use of the sintered body of the fine particle of calcium phosphate allows the fine particle of calcium phosphate to take prominent effects of improving the collagen production promoting action in comparison to unsintered one. Furthermore, the sintered body of the fine particle of calcium phosphate has higher crystallinity and lower solubility in a living body in comparison to amorphous calcium phosphate. Accordingly, the sintered body of the fine particle of calcium phosphate can maintain bioactivity for a long time in a living body, and thus easily exerts effects of promoting collagen production for a long time. The sintered body of the fine particle of calcium phosphate is obtained by sintering amorphous calcium phosphate. Specifically, the sintered body of the fine particle of calcium phosphate is obtained by the sintering with, for example, the method described below. In addition, a fine particle of calcium phosphate having high crystallinity is suitably used. The degree of the crystallinity of this calcium phosphate may be measured by X-ray diffraction (XRD). Specifically, the crystallinity is higher as much as the half maximum full-width of the peak representing each crystal face is narrower. Here, the "highly crystallinity" according to the highly crystalline calcium phosphate of the present invention is 0.8 or less (suitably, 0.5 or less) of the half maximum full-width at d = 2.814.

The breast enlargement promoter according to the present best embodiment suitably contains other breast enlargement-promoting active ingredients, alcohols, sugars, proteins, amino acids, water-soluble vitamins, fat-soluble vitamins, lipids, mucopolysaccharides or a surfactant.

The other breast enlargement-promoting active ingredient to be contained is suitably a collagen peptide, an extract of Pueraria mirifica root, palmitoyl isoleucine, palmitoyl glycine, cocoyl alanine Na, palmitoyl dipeptide-5 diaminobutyroyl hydroxythreonine alanine, palmitoyl dipeptide-5 diaminohydroxybutyric acid, porphyrin or isoflavones.

Examples of the alcohols include, for example, ethanol, and glycerin. The alcohol contained improves the dispersion stability of the fine particle of calcium phosphate, and encourages rapid permeation into the dermic layer. In addition, the alcohol exerts skin washing, astringent and moisturizing effects. Examples of the sugars include, for example, hydrolyzed hydrated starch. The sugar contained improves the moisturizing effects and the anti-inflammatory effects. Examples of the proteins include, for example, collagen. The protein contained acts as a carrier of calcium phosphate and can encourage the delivery into the dermic layer. Examples of the amino acids include, for example, glutamic acid, arginine acid, or a sodium salt thereof. The amino acid contained can encourage collagen production. Examples of the water-soluble vitamins include, for example, magnesium ascorbyl phosphate. The water-soluble vitamins contained can promote collagen production, and can be expected to exert synergistic effects with calcium phosphate. Examples of the fat-soluble vitamins include, for example, vitamin A. The fat-soluble vitamins contained can promote production of mucopolysaccharides and give moisture to the skin. Examples of the lipid include, for example, ceramide and phospholipid. The lipid contained takes moisturizing effects. Examples of the mucopolysaccharides include, for example, hyaluronic acid. The mucopolysaccharides contained take moisturizing effects. Examples of the surfactant include, for example, polyethylene glycol and stearic acid derivatives. The surfactant contained improves dispersion of the fine particle and a fat-soluble component such as vitamin A.

The blending amount of the fine particle of calcium phosphate in the breast enlargement promoter according to the present best embodiment may be suitably adjusted, but is suitably 0.01 to 30% by mass more suitably 0.1 to 15% by mass, and even more suitably 0.1 to 10% by mass with reference to the total mass of the breast enlargement promoter. The blending amount of the other breast enlargement-promoting active ingredient is not particularly limited, but is suitably 0.01 to 30% by mass, more suitably 0.1 to 15% by mass, and even more suitably 0.1 to 10% by mass with reference to the total mass of the breast enlargement promoter. The blending amount of the alcohols is not particularly limited, but is suitably 0.01 to 70% by mass, more suitably 0.01 to 50% by mass, and even more suitably 0.01 to 30% by mass with reference to the total mass of the breast enlargement promoter. The blending amount of the sugars is not particularly limited, but is suitably 0.01 to 70% by mass, more suitably 0.01 to 50% by mass, and even more suitably 0.01 to 30% by mass with reference to the total mass of the breast enlargement promoter. The blending amount of the proteins is not particularly limited, but is suitably 0.01 to 30% by mass, more suitably 0.01 to 20% by mass, and even more suitably 0.01 to 10% by mass with reference to the total mass of the breast enlargement promoter. The blending amount of the amino acids is not particularly limited, but is suitably 0.01 to 30% by mass, more suitably 0.01 to 20% by mass, and even more suitably 0.01 to 10% by mass with reference to the total mass of the breast enlargement promoter. The blending amount of the water-soluble vitamins is not particularly limited, but is suitably 0.001 to 30% by mass, more suitably 0.001 to 20% by mass, and even more suitably 0.001 to 10% by mass with reference to the total mass of the breast enlargement promoter. The blending amount of the fat-soluble vitamins is not particularly limited, but is suitably 0.001 to 30% by mass, more suitably 0.001 to 20% by mass, and even more suitably 0.001 to 10% by mass with reference to the total mass of the breast enlargement promoter. The blending amount of the lipid is not particularly limited, but is suitably 0.001 to 30% by mass, more suitably 0.001 to 20% by mass, and even more suitably 0.001 to 10% by mass with reference to the total mass of the breast enlargement promoter. The blending amount of the mucopolysaccharides is not particularly limited, but is suitably 0.01 to 70% by mass, more suitably 0.01 to 50% by mass, and even more suitably 0.01 to 30% by mass with reference to the total mass of the breast enlargement promoter. The blending amount of the surfactant is not particularly limited, but is suitably 0.001 to 30% by mass, more suitably 0.001 to 20% by mass, and even more suitably 0.001 to 10% by mass with reference to the total mass of the breast enlargement promoter.

### <Manufacture method>

Next, a method of manufacturing the fine particle of calcium phosphate according to the present best embodiment will be explained. The fine particle of calcium phosphate may be artificially manufactured by a known manufacture method such as a wet method, a dry method, a hydrolysis method and a hydrothermal method, or may be naturally derived from the bone, the tooth and the like. In addition, a calcium phosphate particle having a big particle size may be manufactured, followed by being pulverized by a widely known method.

The fine particle of calcium phosphate is suitably obtained by sintering amorphous calcium phosphate. In addition, the lower limit value of the sintering temperature is more preferably 500°C or more. If the sintering temperature is lower than 500°C, the sintering may not be sufficient. On the other hand, the upper value of the sintering temperature is more preferably 1800°C or less, more preferably 1250°C or less, and particularly preferably 1200°C or less. If the sintering temperature is higher than 1800°C, the calcium phosphate may be decomposed. Accordingly, the sintering temperature within the range described above makes it possible to manufacture poorly soluble (high crystallinity) calcium phosphate in a living body. In addition, the sintering time is not particularly limited, but may be suitably established. Meanwhile, the particles may be fused to each other by the sintering, but in such a case, the particles may be pulverized and used after the sintering.

The fine particles of the calcium phosphate according to the present best embodiment are particularly suitably manufactured by the method described below. Specifically, the method of manufacturing the fine particle of calcium phosphate according to the present best embodiment is suitably a method that includes a dispersion firing (sintering) method including at least one of a mixing process and a sintering process. The fine particle obtained by the dispersion firing method reflects the particle size of the primary particle as it is, which makes it easy to prepare the average particle size in the range described above. In addition, the manufacture method according to the present best embodiment may include primary particle production process, and removal process. These processes are performed in the order of, for example, the primary particle production process, the mixing process, the sintering process, and the removal process.

### (Primary particle production process)

The primary particle production process is not particularly limited as long as it is a process that allows production of the fine particle of calcium phosphate, and the primary particle production process may be suitably selected, and adopted depending on a raw material of highly crystalline fine particle of calcium phosphate to be manufactured. For example, if phosphoric acid is dropped to a slurry of calcium hydroxide under normal temperature, particles of calcium phosphate (CaP) are precipitated.

A method of producing a group of primary particles that are minute (nanometer size) and have uniform particle size (the particle size distribution is narrow) such as the fine particle of calcium phosphate according to the present best embodiment, is not particularly limited, but for example, the method described in JP-A No. 2002-137910 may be used. Namely, the fine particle (primary particle) of calcium phosphate (hydroxyapatite) may be synthesized by solubilizing and mixing a calcium solution and a phosphoric acid solution in the surfactant/water/oil-based emulsion phase, and reacting the mixture at the clouding point of the surfactant or more. In addition, at this time, it is possible to control the size of the fine particle of calcium phosphate by changing the ratio of the hydrophilic group/hydrophobic group and the functional group described above of the surfactant.

The principle for manufacturing the fine particle of calcium phosphate described above will be explained below, briefly. In a method of synthesizing the fine particle of calcium phosphate by solubilizing the calcium solution and the phosphoric acid solution in the surfactant/water/oil-based emulsion phase, and mixing and reacting them, the nucleus of calcium phosphate is grown in the micelle of the surfactant, and the particle is grown. At this time, it is possible to control the thermodynamic stability of the micelle by changing the reaction temperature (when the surfactant is a nonionic surfactant, the reaction temperature is set up to the clouding point of the surfactant or higher). Namely, elevation of the reaction temperature is reduction of the force to form the micelle of the surfactant. Then, it is considered that the driving force for growing the particle of calcium phosphate that is limited in the frame of the micelle becomes greater than the driving force for maintaining the frame of the micelle. Accordingly, it is possible to control the shape of the particle with use of the mechanism.

In manufacture of the micelle of the surfactant, the functional group (hydrophilic site) of the surfactant, and the ratio of the hydrophilicity/hydrophobicity in the molecule are important, and the stability and the clouding point of the micelle depend on them. In addition, the clouding point of the surfactant depends on the kind. Therefore, it is possible to change the functional group and the ratio of hydrophilicity/hydrophobicity of the surfactant described above, and control the size of the fine particle of calcium phosphate by suitably changing the kind of the surfactant.

Meanwhile, the kind of the surfactant used in the method described above is not particularly limited, and may be suitably selected and used from the other kinds of known anionic, cationic, zwitterion and nonionic surfactants disclosed in JP-A No. HEI-5-17111 described above. When the nonionic surfactant is used among the surfactants, control of the shape of the crystal using the mechanism described above becomes easy since the surfactant has the clouding point. More specifically, as the nonionic surfactant, use can be made of polyoxyethylene alkyl ether, polyoxyethylene allyl ether, polyoxyethylene alkylallyl ether, polyoxyethylene derivatives, oxyethylene/oxypropylene block copolymer, sorbitan fatty acid ester, polyoxyethylene sorbitan fatty acid ester, polyoxyethylene sorbitol fatty acid ester, glycerin fatty acid ester, polyoxyethylene fatty acid ester, polyoxyethylene alkyl amine and the like. In addition, as the cationic surfactant, use can be made of quaternary ammonium salts such as stearyl amine hydrochloride, lauryl trimethyl ammonium chloride, and alkylbenzene dimethyl ammonium chloride. As the anionic surfactant, use can be made of higher alcohol sulfuric acid ester salts such as sodium lauryl alcohol sulfuric acid ester and sodium oleyl alcohol sulfuric acid ester, alkylsulfuric acid salts such as sodium lauryl sulfate and ammonium lauryl sulfate, alkylaryl sulfonic acid salts such as sodium dodecyl benzene sulfonate, and sodium dodecyl naphthalene sulfonate. As the zwitterion surfactant, use can be made of an alkyl betaine type, an alkyl amide betaine type, an amine oxide type and the like. The surfactants described above are used in one kind, or in combination of two or more kinds. Among them, pentaethylene glycol dodecyl ether is particularly desirably used from the point of the clouding point and the solubility.

In addition, examples of the oil phase that may be used in the method described above include, for example, hydrocarbons such as toluene, xylene, hexane, dodecane and cyclohexane, halogenated hydrocarbons such as chlorobenzene and chloroform, ethers such as diethyl ether, alcohols such as butanol, and ketones such as methyl isobutyl ketone and cyclohexanone. These solvents are selected in one kind or two kinds such that they have small water solubility, and dissolve any one of the surfactants described above depending on the surfactant to be used. Among them, dodecane is particularly desirably used from the point of the water solubility and the solubility of the surfactant. Other conditions such as the reaction temperature, the reaction time, and the addition amount of the raw material may be selected and adopted suitably to be optimal depending on the composition of the primary particles. However, the upper limit of the reaction temperature is preferably a temperature where the solution does not boil, preferably 90°C or less since the reaction is of a water solution.

In addition, the present process may include a process of washing the produced primary particles with water and the like, and processes of centrifuging and recovering the primary particles with filtration or the like.

### (Mixing process)

The mixing process is a process of mixing the primary particle and the fusion inhibitor. With the fusion inhibitor to be rendered in advance to intervene between the particles of the primary particle group obtained by the primary particle production process described above, it is possible to prevent the primary particles from fusion to each other in the following sintering process. Meanwhile, a mixture of the primary particle and the fusion inhibitor obtained by the mixing process is referred to as the "mixed particle".

Here, the "fusion inhibitor" is not particularly limited as long as it can prevent fusion between the primary particles, but is preferably non-volatile at the sintering temperature of the following sintering process. This is because the fusion inhibitor does not disappear between the primary particles due to the non-volatility under the condition of the sintering temperature, and thus fusion of the primary particles to each other can be surely prevented during the sintering process. However, the fusion inhibitor is not necessarily 100% non-volatile at the sintering temperature, but may be non-volatile such that 10% or more of the fusion inhibitor remains between the primary particles after completion of the sintering process. In addition, the fusion inhibitor may be chemically decomposed by heat after completion of the sintering process. Namely, if the fusion inhibitor remains after completion of the sintering process, the fusion inhibitor is not necessarily the same substance (compound) before and after initiation of the sintering process.

In addition, the fusion inhibitor is preferably a substance that dissolves in a solvent, particularly, a water-based solvent. With use of a fusion inhibitor that dissolves in the solvent as the fusion inhibitor as described above, it is possible to remove the fusion inhibitor (for example, calcium carbonate and the like) by merely suspending the fine particles of calcium phosphate mixed with the fusion inhibitor in a water-based solvent such as pure water. Particularly, in the case of the fusion inhibitor that dissolves in a water-based solvent, there is no need to use an organic solvent when removing the fusion inhibitor, and thus an equipment corresponding to use of the organic solvent in the removal process, or waste liquid treatment of the organic solvent becomes unnecessary. Consequently, it is possible to remove the fusion inhibitor more conveniently from the fine particle of calcium phosphate. The solvent described above is not particularly limited, but examples thereof include, for example, water, ethanol, and methanol as a water-based solvent, and acetone, and toluene as the organic solvent.

In addition, the water-based solvent described above may contain a chelate compound such as oxalic acid salt, ethylene diamine, bipyridine and ethylenediamine tetraacetate in order to elevate the solubility of the fusion inhibitor in water. Furthermore, the water-based solvent described above may contain an electrolytic ion such as sodium chloride, ammonium nitrate, and potassium carbonate in order to elevate the solubility of the fusion inhibitor in water.

Here, it can be said that the solubility of the fusion inhibitor with respect to the solvent is preferably high since higher solubility allows higher removal efficiency. The preferable solubility to be concerned is preferably 0.01 g or more, more preferably 1 g or more, and most preferably 10 g or more as the solubility of the amount (g) of the solute with respect to 100 g of the solvent.

Specific examples of the fusion inhibitor described above include calcium salts (or complex) such as calcium chloride, calcium oxide, calcium sulfate, calcium nitrate, calcium carbonate, calcium hydroxide, calcium acetate and calcium citrate, potassium salts such as potassium chloride, potassium oxide, potassium sulfate, potassium nitrate, potassium carbonate, potassium hydroxide and potassium phosphate, and sodium salts such as sodium chloride, sodium oxide, sodium sulfate, sodium nitrate, sodium carbonate, sodium hydroxide and sodium phosphate.

Meanwhile, a method for mixing the primary particle and the fusion inhibitor in the mixing process is not particularly limited, and may be a method of mixing the solid of the fusion inhibitor with the solid of the primary particles, and then mixing them using a blender. Alternatively, a method may be also performed, in which the primary particles are dispersed in a solution of the fusion inhibitor. However, it can be said that the latter is a preferable method in order to render the fusion inhibitor to uniformly and surely intervene between the primary particles due to the difficulty in uniform mixing between the solids. When the latter method is adopted, a solution of the fusion inhibitor in which the primary particles are dispersed, is preferably kept dry. This is because it is possible to keep the uniformly mixed state of the primary particles and the fusion inhibitor for a long time. Also in Examples described below, 0.5 g of hydroxyapatite (HAp) primary particle is dispersed in a saturated aqueous solution of calcium carbonate, and dried at 80°C whereby to obtain the mixed particle.

In addition, the mixing process may be a process in which a solution containing a high molecular compound having any one of a carboxyl group, a sulfuric acid group, a sulfonic acid group, a phosphoric acid group, a phosphonic acid group, an amino group or a salt thereof on the side chain, is mixed with the primary particle described above, and a metal salt (alkaline metal salt and/or alkaline earth metal salt and/or transitional metal salt) is further added. By adopting the process described above, the high molecular compound adsorbs onto the surface of calcium phosphate {hydroxyapatite (HAp)}, which makes it possible to surely prevent contact of calcium phosphate {hydroxyapatite (HAp)} to each other in the process of mixing the fusion inhibitor, and the following addition of the calcium salt makes it possible to precipitate surely the fusion inhibitor on the surface of calcium phosphate {hydroxyapatite (HAp)}. Meanwhile, the high molecular compound having any one of a carboxyl group, a sulfuric acid group, a sulfonic acid group, a phosphoric acid group, a phosphonic acid group, an amino group or a salt thereof on the side chain is simply referred to as the "high molecular compound" in the description below.

The high molecular compound described above is not particularly limited as long as it is a compound having any one of a carboxyl group, a sulfuric acid group, a sulfonic acid group, a phosphoric acid group, a phosphonic acid group, an amino group or a salt thereof on the side chain. For example, examples of the high molecular compound having a carboxyl group on the side chain include polyacrylic acid, polymethacrylic acid, sodium polyacrylate, sodium polymethacrylate, carboxymethyl cellulose, and a styrene-maleic anhydride copolymer. Examples of the high molecular compound having a sulfuric acid group on the side chain include polyacrylic acid alkylsulfuric acid ester, polymethacrylic acid alkylsulfuric acid ester, and polystyrene sulfuric acid. Examples of the high molecular compound having a sulfonic acid group on the side chain include polyacrylic acid alkylsulfonic acid ester, polymethacrylic acid alkylsulfonic acid ester, and polystyrene sulfuric acid. Examples of the high molecular compound having a phosphoric acid group on the side chain include polyacrylic acid alkylphosphoric acid ester, polymethacrylic acid alkylphosphoric acid ester, polystyrene phosphoric acid, and polyacryloylaminomethyl phosphonic acid. Examples of the high molecular compound having a phosphonic acid group on the side chain include polyacrylic acid alkylphosphonic acid ester, polymethacrylic acid alkylphosphonic acid ester, polystyrene phosphonic acid, polyacryloylaminomethyl phosphonic acid, and polyvinylalkylphosphonic acid. Examples of the high molecular compound having an amino group on the side chain include polyacrylic amide, polyvinyl amine, polymethacrylic acid aminoalkyl ester, polyaminostyrene, a polypeptide, and a protein. Meanwhile, any one of the high molecular compounds described above may be used in one kind, or multiple kinds of the high molecular compounds may be mixed in the mixing process.

Meanwhile, the molecular weight of the high molecular compound described above is not particularly limited, but preferably 100 g/mol or more and 1,000,000 g/mol or less, more preferably 500 g/mol or more and 500, 000 g/mol or less, and most preferably 1,000 g/mol or more and 300,000 g/mol or less. If the molecular weight of the high molecular compound is less than the preferable range described above, the intervening ratio between the primary particles decreases, and the ratio of the inhibition of the contact of the primary particles to each other decreases. In addition, if the molecular weight of the high molecular compound is more than the preferable range described above, the operability becomes worse such as decrease of the solubility of the high molecular compound, and increase of the viscosity of the solution containing the high molecular compound, which is not preferable.

Meanwhile, the solution containing the high molecular compound is preferably an aqueous solution. This is because the sintered body particle of calcium phosphate {hydroxyapatite (HAp)} dissolves under a strong acidic condition. Meanwhile, the pH of the aqueous solution containing the high molecular compound is not particularly limited as long as it is a condition where the HAp particle is insoluble at 5 or more and 14 or less of the pH. The aqueous solution containing the high molecular compound may be obtained by dissolving the high molecular compound in distilled water, ion exchange water and the like, and adjusting the pH with an aqueous solution of ammonia, an aqueous solution of sodium hydroxide or potassium hydroxide, or the like.

In addition, the concentration of the high molecular compound contained in the aqueous solution described above is preferably 0.001% w/v or more and 50% w/v or less, more preferably 0.005% w/v or more and 30% w/v or less, and most preferably 0.01% w/v or more and 10% w/v or less. If the concentration of the high molecular compound contained in the aqueous solution is less than the preferable range described above, the intervening amount between the primary particles is small, and the ratio of the inhibition of the contact of the primary particles to each other decreases. In addition, if the concentration of the high molecular compound contained in the aqueous solution is more than the preferable range described above, the operability becomes worse such as poor solubility of the high molecular compound, and increase of the viscosity of the solution containing the high molecular compound, which is not preferable.

In the mixing process in the present invention, the solution containing the high molecular compound described above is mixed with the primary particles. Such mixing may be performed by, for example, inputting the primary particles into the solution, and dispersing the primary particles by stirring operation and the like. By such operation in the method of manufacturing calcium phosphate according to the present invention described above, it is possible for the high molecular compound described above to adsorb on the surface of the primary particle, and add any one of a carboxyl group, a sulfuric acid group, a sulfonic acid group, a phosphoric acid group, a phosphonic acid group, an amino group or a salt thereof on the surface of the primary particle. At this time, the carboxyl group, the sulfuric acid group, the sulfonic acid group, the phosphoric acid group, the phosphonic acid group or the amino group is present as the state of ion in the solution.

Next, to a solution in which the solution containing the high molecular compound is mixed with the primary particle, a metal salt (alkaline metal salt and/or alkaline earth metal salt and/or transitional metal salt) is further added, which makes the carboxylic acid ion, the sulfuric acid ion, the sulfonic acid ion, the phosphoric acid ion, the phosphonic acid ion, or the amino ion present on the surface of the primary particle described above bind to the metal ion (alkaline metal ion and/or alkaline earth metal ion and/or transitional metal ion), and generates the carboxylic acid salt, the sulfuric acid salt, the sulfonic acid salt, the phosphoric acid salt, the phosphonic acid salt, or the amino acid salt on the surface of the primary particle. Such carboxylic acid salt, sulfuric acid salt, sulfonic acid salt, phosphoric acid salt, phosphonic acid salt, or amino acid salt of the metal (alkaline metal and/or alkaline earth metal and/or transitional metal) functions as the fusion inhibitor described above. Accordingly, the primary particle in which the carboxylic acid salt, sulfuric acid salt, sulfonic acid salt, phosphoric acid salt, phosphonic acid salt, or amino acid salt of the metal (alkaline metal and/or alkaline earth metal and/or transitional metal) is generated on the surface is so-called the "mixed particle". Meanwhile, such carboxylic acid salt, sulfuric acid salt, sulfonic acid salt, phosphoric acid salt, phosphonic acid salt, or amino acid salt of the metal (alkaline metal and/or alkaline earth metal and/or transitional metal) is precipitated, and thus the precipitated substance may be recovered, and then dried, and subjected to the sintering process described below. Examples of the drying include, for example, a method in which drying is performed by heating (preferably 0°C or more and 200°C or less, more preferably 20°C or more and 150°C or less, and most preferably 40°C or more and 120°C or less) under the condition of reduced pressure (preferably 1 × 10⁵ Pa or more and 1 × 10⁻⁵ Pa or less, more preferably 1 × 10³ Pa or more and 1 × 10⁻³ Pa or less, and most preferably 1 × 10² Pa or more and 1 × 10⁻² Pa or less). Meanwhile, in the drying described above, the drying is preferably under the condition of reduced pressure since it can lower the dry temperature, but the drying may be also performed under the condition of atmospheric pressure.

The alkaline metal salt as described above is not particularly limited, but use can be made of, for example, sodium chloride, sodium hypochlorite, sodium chlorite, sodium bromide, sodium iodide, sodium iodate, sodium oxide, sodium peroxide, sodium sulfate, sodium thiosulfate, sodium selenate, sodium nitrite, sodium nitrate, sodium phosphate, sodium carbonate, sodium hydroxide, potassium chloride, potassium hypochlorite, potassium chlorite, potassium bromide, potassium iodide, potassium iodate, potassium oxide, potassium peroxide, potassium sulfate, potassium thiosulfate, potassium selenate, potassium nitrite, potassium nitrate, potassium phosphate, potassium carbonate, potassium hydroxide and the like.

In addition, as the alkaline earth metal salt described above, use can be made of, for example, magnesium chloride, magnesium hypochlorite, magnesium chlorite, magnesium bromide, magnesium iodide, magnesium iodate, magnesium oxide, magnesium peroxide, magnesium sulfate, magnesium thiosulfate, magnesium selenate, magnesium nitrite, magnesium nitrate, magnesium phosphate, magnesium carbonate, magnesium hydroxide, calcium chloride, calcium hypochlorite, calcium chlorite, calcium bromide, calcium iodide, calcium iodate, calcium oxide, calcium peroxide, calcium sulfate, calcium thiosulfate, calcium selenate, calcium nitrite, calcium nitrate, calcium phosphate, calcium carbonate, calcium hydroxide and the like.

In addition, as the transitional metal salt described above, use can be made of, for example, zinc chloride, zinc hypochlorite, zinc chlorite, zinc bromide, zinc iodide, zinc iodate, zinc oxide, zinc peroxide, zinc sulfate, zinc thiosulfate, zinc selenate, zinc nitrite, zinc nitrate, zinc phosphate, zinc carbonate, zinc hydroxide, iron chloride, iron hypochlorite, iron chlorite, iron bromide, iron iodide, iron iodate, iron oxide, iron peroxide, iron sulfate, iron thiosulfate, iron selenate, iron nitrite, iron nitrate, iron phosphate, iron carbonate, iron hydroxide and the like. In addition, the transitional metal salt may be a nickel compound.

Meanwhile, the metal salt (alkaline metal salt, alkaline earth metal salt, transitional metal salt) that is added to the solution in which the solution containing the high molecular compound is mixed with the primary particles may be in one kind, or may be a mixture of 2 or more kinds. In addition, the metal salt (alkaline metal salt, alkaline earth metal salt, transitional metal) is preferably added as an aqueous solution for the reason that it allows uniform addition, and control of the concentration to be added, and the like although the metal salt may be in the solid state. In addition, the amount (concentration) of the metal salt (alkaline metal salt and/or alkaline earth metal salt and/or transitional metal salt) to be added is not particularly limited as long as it is a condition where the metal salt binds to a carboxylic acid ion, a sulfuric acid ion, a sulfonic acid ion, a phosphoric acid ion, a phosphonic acid ion, or an amino ion present on the surface of the primary particle, whereby to generate a carboxylic acid salt, a sulfuric acid salt, a sulfonic acid salt, a phosphoric acid salt, a phosphonic acid salt, or an amino acid salt of the metal (alkaline metal and/or alkaline earth metal and/or transitional metal), and may be with suitably studied and determined.

Meanwhile, the carboxylic acid salt, the sulfuric acid salt, the sulfonic acid salt, the phosphoric acid salt, the phosphonic acid salt, or the amino acid salt of the metal (alkaline metal and/or alkaline earth metal and/or transitional metal) generated on the surface of the primary particle by the process described above receives thermal decomposition in the sintering process described below to become an oxide of the metal (alkaline metal and/or alkaline earth metal and/or transitional metal). For example, in a case where calcium polyacrylate is generated on the surface of the primary particle, the calcium polyacrylate becomes calcium oxide by the sintering process. Meanwhile, since the metal oxide (alkaline metal oxide and/or alkaline earth metal oxide (for example, calcium oxide) and/or transitional metal oxide) is water-soluble, it may be easily removed by the removal process described below.

Meanwhile, sodium polyacrylate is soluble in water, and thus may be used as it is as the fusion inhibitor in the present mixing process. However, calcium polyacrylate is insoluble in water, and thus it is preferable that first, polyacrylic acid only is adsorbed on the surface of the primary particle, and then calcium salt and the like are added, whereby to precipitate calcium polyacrylate on the surface of the primary particle. In addition, the high molecular compound is decomposed when the primary particle is calcinated at high temperature (about 300°C or more), and thus it can be said that a metal salt of the high molecular compound is preferably allowed to be precipitated on the surface of the primary particle so as to function as the fusion inhibitor after the calcination. However, in a case where the primary particle is calcinated (heat treatment) at a temperature where high molecular compound is not decomposed (not softened), the metal salt of the high molecular compound is not particularly necessarily kept precipitated on the surface of the primary particle.

### (Sintering process)

The sintering process is a process in which the mixed particle obtained by the mixing process described above is exposed to a sintering temperature, and the primary particle contained in the mixed particle is made to a highly crystalline fine particle of calcium phosphate (particle of a sintered body). Due to the fusion inhibitor intervening between the particles of the primary particles, fusion of the primary particles to each other can be prevented even if the primary particles are exposed to the high temperature condition in the sintering process.

The sintering temperature in the sintering process may be suitably established so that the hardness of the highly crystalline fine particle of calcium phosphate becomes a desired hardness, and is, for example, more preferably in a range of 100°C to 1800°C, more preferably 150°C to 1500°C, and most preferably 200°C to 1200°C. Meanwhile, the sintering time may be suitably established based on the desired hardness of the highly crystalline fine particle of calcium phosphate, and the like. The sintering is performed at 800°C for 1 hour in Examples described below.

Meanwhile, a device and the like used in the sintering process are not particularly limited, but commercially available firing furnace may be suitably selected and adopted depending on the manufacture scale, manufacture conditions and the like.

### (Removal process)

The removal process is a process of removing the fusion inhibitor mixed between the particles of highly crystalline fine particles of calcium phosphate obtained by the sintering process.

Means and a method for the removal may be suitably adopted depending on the fusion inhibitor adopted in the mixing process described above. For example, in a case where a fusion inhibitor having solvent solubility is used, the fusion inhibitor can be removed by dissolving only the fusion inhibitor using a solvent that does not dissolve (insoluble) the fine particle of calcium phosphate, but dissolves (soluble) the fusion inhibitor. The solvent to be used is not particularly limited as long as it is a solvent that meets the requirement described above, and may be a water-based solvent, or an organic solvent. For example, examples of the solvent include water, ethanol, and methanol as the water-based solvent, and acetone, and toluene as the organic solvent.

In addition, the water-based solvent described above may contain a chelate compound such as oxalic acid salt, ethylene diamine, bipyridine, and ethylenediamine tetraacetate in order to elevate the solubility of the fusion inhibitor in water. Furthermore, the water-based solvent described above may contain an electrolytic ion such as sodium chloride, ammonium nitrate, and potassium carbonate in order to elevate the solubility of the fusion inhibitor in water.

However, the solvent to be used is preferably a water-based solvent for the reasons that an equipment corresponding to use of an organic solvent is unnecessary in the removal process, that waste liquid treatment of an organic solvent is unnecessary, that the safety of the manufacture operation is high, and that the risk with respect to the environment is low.

Meanwhile, in a case of the sintered body particle of highly crystalline calcium phosphate {hydroxyapatite (HAp)}, the removal process is preferably performed at pH 4.0 to pH 12.0 since the sintered body particle of highly crystalline calcium phosphate {hydroxyapatite (HAp)} dissolves under the condition of pH 4.0 or less.

Incidentally, in a case where the fusion inhibitor is removed using a solvent, the removal process may be such that calcium phosphate containing the fusion inhibitor obtained by the sintering process is suspended in the solvent, and then only the calcium phosphate particle is recovered by filtration or centrifuge. The operation described above in the method of manufacturing calcium phosphate according to the present best embodiment, is not limited to one time, but may be performed twice or more times. With performing multiple times of the operation described above, it can be said that the removal rate of the fusion inhibitor of calcium phosphate is further improved. However, the operation described above is preferably not performed more than necessary for the reasons that the manufacture process becomes complicated, that the manufacture cost increases, that the recovery rate of calcium phosphate decreases, and the like. Accordingly, the number of times of the operation described above may be suitably determined based on a targeted removal rate of the fusion inhibitor.

Meanwhile, the present process may encompass a classification process in order to further make the particle size uniform.

In addition to the method of removing the fusion inhibitor using the solvent described above, the fusion inhibitor may use a magnetic material, and be removed using a magnet. More specifically, a group of the calcium phosphate particles (coarse calcium phosphate particle) containing the fusion inhibitor obtained by the sintering process may be suspended and dispersed in an appropriate solvent (water and the like), and then the suspension is applied with the magnetic force, and only the fusion inhibitor is adsorbed on the magnet, and only the calcium phosphate particle not adsorbed is recovered. In addition, particularly, a method may be performed without suspension in a solvent, in which a coarse calcium phosphate particle may be triturated to powders, and then the fusion inhibitor may be isolated by the magnet. However, it can be said that the method in which the calcium phosphate particle is made to a suspension allows easy separation of the calcium phosphate particle and the fusion inhibitor, and higher removal rate of the fusion inhibitor. Meanwhile, the calcium phosphate particle to which this method may be applied, is preferably a non-magnetic material or a weak magnetic material.

### <Properties>

The calcium phosphate particle manufactured by the method of manufacturing the calcium phosphate particle according to the present best embodiment described above, keeps the state of the primary particle in the majority since the action of the fusion inhibitor prevents the fusion of the primary particles to each other. Accordingly, when the highly crystalline calcium phosphate particle is suspended in a solvent, the highly crystalline calcium phosphate particle may be dispersed as a primary particle in which the majority of the highly crystalline calcium phosphate particles contains a single crystal, or a particle mass (single crystal primary particle) in which the primary particles containing the single crystal are collected with ionic interaction.

The calcium phosphate particle according to the present best embodiment is a primary particle of which the majority contains a single crystal, or a particle mass in which the primary particles containing the single crystal are collected with ionic interaction (single crystal primary particle), and has good dispersion in a solvent, and also has high surface area due to no formation of a secondary particle.

Here, a method of evaluating whether the fine particle of calcium phosphate is present as a primary particle, or not, is, for example, a method in which measurement results of the particle size by observation with an electronic microscope are compared with measurement results of the particle size in the state as suspended in a solvent by dynamic light scattering method. If the two results are consistent with each other, it can be judged that most of the calcium phosphate particles according to the present best embodiment are in the state of the primary particle. In addition, if the measure result of the particle size by observation with an electronic microscope is greater than the measure result of the particle size by the dynamic light scattering method, it can be judged that fusion of the primary particles to each other occurs to form a secondary particle.

Meanwhile, the solvent in which the calcium phosphate particle according to the present best embodiment is dispersed, is not particularly limited as long as it does not dissolve the calcium phosphate particle. Examples of the solvent include, for example, water, alcohols such as methanol and ethanol, ketones such as acetone, methyl ethyl ketone, methyl isobutyl ketone and cyclohexanone, amides such as N,N-dimethyl form amide, sulfoxides such as dimethyl sulfoxide, hydrocarbons such as toluene, xylene, hexane, dodecane and cyclohexane, halogenated hydrocarbons such as chlorobenzene and chloroform, and ethers such as diethyl ether and dioxane. These solvents may be selected and used in one kind or two kinds depending on the purpose of the use.

The ratio of the primary particle containing the single crystal, or the particle mass in which the primary particles containing the single crystal are collected with ionic interaction (single crystal primaryparticle) may be calculated by obtaining the particle size distribution diagram obtained from the dynamic light scattering method, and obtaining the ratio of the particles of which the particle size is almost consistent with the particle size of the primary particle obtained from an electronic microscope.

Meanwhile, according to the method of manufacturing the highly crystalline calcium phosphate particle according to the present best embodiment described above, at least 50% or more, and more suitably 60% or more of the calcium phosphate particle can be present as the single crystal primary particle, and 70% or more of the calcium phosphate particle under the most suitable condition can be present as the single crystal primary particle, although it may vary depending on a raw material for calcium phosphate, the kind of the fusion inhibitor, the sintering conditions and the like.

### (Dosage regimen)

The method of promoting breast enlargement by applying the breast enlargement promoter according to the present best embodiment to the breast is not particularly limited. For example, the breast enlargement promoter exerts the efficacy by being used topically once to three times (suitably twice), 250 to 4000 mg per time (suitably, 500 to 2000 mg, even more suitably 1000 mg) (however, calcium phosphate is contained in 10 to 300 mg (suitably 25 to 200 mg, more suitably 50 mg) in this) per day to be applied to the breast (particularly the breast on the upper side). Furthermore, the breast enlargement promoter exerts much further efficacy with continuous use for a long time of 1 week to 1 year.

### Examples

### Manufacture Example 1 (Manufacture of the fine particle of calcium phosphate)

### (Primary particle production process)

Dodecane [CH₃(CH₂)₁₀CH₃] was used as a continuous oil phase, and pentaethylene glycol dodecyl ether [CH₃(CH₂)₁₀CH₂O(CH₂CH₂O)₄CH₂CH₂OH] having 31°C of the clouding point was used as a nonionic surfactant. 40 ml of the continuous oil phase containing 0. 5 g of the nonionic surfactant described above was prepared at room temperature. Next, 10 ml of 2.5 mol/l calcium hydroxide [Ca(OH)₂] dispersed-aqueous solution was added to the continuous oil phase prepared described above, to prepare a water in oil type solution (W/O solution). 10 ml of 1.5 mol/l potassium dihydrogen phosphate [(KH₂PO₄)] solution was added to the W/O solution described above while stirring the solution. Then, the reaction solution was allowed to react for 24 hours at room temperature while being stirred. Next, the obtained reactant was isolated by centrifuge and washed, whereby to obtain a group of the hydroxyapatite (HAp) primary particles.

### (Mixing process)

To 100 ml aqueous solution of pH 12.0 containing 1.0 g of sodium polyacrylate (15,000 g/mol weight average molecular weight, manufactured by ALDRICH), 1.0 g of the group of the hydroxyapatite (HAp) primary particles was dispersed, whereby to adsorb sodium polyacrylate on the surface of the same particle. The pH of this aqueous solution was measured using a pH meter D-24 SE manufactured by HORIBA, Ltd.

Next, to the dispersion prepared described above, 100 ml of 0.12 mol/l aqueous solution of calcium nitrate [Ca(NO₃)₂] was added, whereby to precipitate calcium polyacrylate on the surface of the same primary particle. Such calcium polyacrylate is a fusion inhibitor. The resulting precipitate was recovered, and dried at 80°C under reduced pressure (about 0.1 Pa), to obtain the mixed particle.

### (Sintering process)

The mixed particle described above was put in a crucible, and the sintering was performed for one hour at 800°C of the sintering temperature. At this time, calcium polyacrylate was thermally decomposed to give calcium oxide [CaO]. The residual ratio of calcium oxide [CaO] after completion of the sintering process was 25% or more.

### (Removal process)

50 mmol/l aqueous solution of ammonium nitrate [NH₄NO₃] was prepared in order to elevate the solubility of the fusion inhibitor in water. Next, the sintered body obtained in the process described above was suspended in 500 ml of the aqueous solution prepared described above, and isolated by centrifuge and washed. The resultant was further suspended in distilled water, and similarly isolated by centrifuge and washed whereby to remove the fusion inhibitor and ammonium nitrate, and recover the highly crystalline fine particles of hydroxyapatite (HAp). Detailed information for the hydroxyapatite fine particle obtained by these processes is summarized below.

Half maximum full-width of XRD: 0.2 (d = 2.814)
Shape: spherical shape
Average particle size (from electronic microscope): 28 nm
Variation coefficient: 14%

### Manufacture Example 2 (unfired)

The primary particle production process only was performed under the same conditions as those of Manufacture Example 1 except that the reaction temperature was set up to 30°C in the primary particle production process, and the following processes such as the mixing process and the sintering process were not performed, whereby to obtain the unfired hydroxyapatite fine particle of Manufacture Example 2. Detailed information for the hydroxyapatite fine particle is described below.

Half maximum full-width of XRD: 0.8(d = 2.814)
Shape: particle shape
Average particle size (from electronic microscope): 42 nm
Variation coefficient: 17%

### <Test for type I collagen production promoting action of fibroblastic cell>

Normal human fibroblastic cells were cultured for 24 hours in a 0.5% FBS-DMEM medium containing the sample, and then the collagen amount in the medium was quantified with ELISA. The experiment was performed 6 times repeatedly in respective conditions (n = 6). Ascorbic acid magnesium phosphate (VCP Mg) was used as a positive control (P.C.). Meanwhile, the used sample is the fired hydroxyapatite manufactured in Manufacture Example 1, and the unfired hydroxyapatite used in Manufacture Example 2. The results are shown in Table 1. Herein, the "mean" in Table 1 represents the measured value (average value) of the obtained, measured collagen amount, and the "SD" represents the standard deviation, and the "p (t-test)" represents the p value obtained by the "t-test" {If the p value is generally 0.05 (0.01 when strictly argued) or less, it represents that the values has difference. Reversely, if the p value is greater than 0. 05, it represents that there is nearly no difference.}.

**[Table 1]**

| Collagen production promoting action of fibroblastic cell (n=6) | | | | | | | | | |
|---|---|---|---|---|---|---|---|---|---|
| Sample | Conc. (µg/mL) | Collagen (µg / mg protein) | | | | Protein (µg / well) | | | |
| | | Mean | ± | SD | ²⁾ p (t-test) | Mean | ± | SD | ²⁾ p (t-test) |
| Manufacture Example 1 Hydroxyapatite (fired) | 0 | 9.84 | ± | 0.40 | 1.000 | 6.80 | ± | 0.38 | 1.000 |
| | ¹⁾ P.C. | 24.28 | ± | 1.06 | 0.000 | 6.99 | ± | 0.56 | 0.516 |
| | 0.80 | 14.99 | ± | 1.43 | 0.000 | 6.65 | ± | 0.55 | 0.603 |
| | 1.60 | 16.69 | ± | 0.75 | 0.000 | 6.67 | ± | 0.23 | 0.484 |
| | 3.10 | 16.66 | ± | 1.07 | 0.000 | 6.62 | ± | 0.42 | 0.443 |
| | 6.30 | 18.06 | ± | 1.12 | 0.000 | 6.84 | ± | 0.31 | 0.846 |
| | 12.50 | 16.94 | ± | 0.53 | 0.000 | 6.60 | ± | 0.25 | 0.310 |
| | 25.00 | 16.93 | ± | 1.30 | 0.000 | 6.63 | ± | 0.26 | 0.379 |
| | 50.00 | 16.17 | ± | 0.96 | 0.000 | 7.03 | ± | 0.26 | 0.256 |
| | 100.00 | 14.29 | ± | 0.38 | 0.000 | 7.08 | ± | 0.34 | 0.209 |
| Manufacture Example 2 Hydroxyapatite (unfired) | 0 | 9.16 | ± | 0.61 | 1.000 | 6.34 | ± | 0.36 | 1.000 |
| | ¹⁾ P.C. | 19.75 | ± | 1.11 | 0.000 | 6.36 | ± | 0.21 | 0.878 |
| | 0.80 | 9.51 | ± | 0.62 | 0.347 | 6.22 | ± | 0.17 | 0.473 |
| | 1.60 | 9.79 | ± | 0.64 | 0.113 | 6.27 | ± | 0.16 | 0.687 |
| | 3.10 | 9.80 | ± | 0.80 | 0.150 | 6.04 | ± | 0.18 | 0.103 |
| | 6.30 | 10.34 | ± | 0.76 | 0.015 | 6.24 | ± | 0.13 | 0.561 |
| | 12.50 | 10.71 | ± | 0.61 | 0.001 | 6.23 | ± | 0.13 | 0.508 |
| | 25.00 | 10.67 | ± | 0.52 | 0.001 | 6.16 | ± | 0.20 | 0.324 |
| | 50.00 | 10.03 | ± | 0.86 | 0.070 | 6.35 | ± | 0.22 | 0.940 |
| | 100.00 | 9.69 | ± | 0.48 | 0.124 | 6.08 | ± | 0.21 | 0.166 |

| | | | | | | | | | |
|---|---|---|---|---|---|---|---|---|---|
| 1) PC : 25 µM VC-PMg 2) represents significant difference with respect to a cell untreated with sample. | | | | | | | | | |

The hydroxyapatite (fired) of Manufacture Example 1 was recognized to have noticeable action of increasing the amount of collagen production. The hydroxyapatite (unfired) of Manufacture Example 2 was also recognized to have increase in significant collagen production amount in the concentration region of 6.30 to 25.00 µg/ml, but had weaker action in comparison with the hydroxyapatite (fired).

### <Test 1 for breast enlargement promotion>

Test conditions: The details and the purpose of the test were explained to subject adult women, and then a patch test in the antebrachial region was performed to check any abnormality, and then the effects of the breast enlargement promoter according to the present invention were investigated.

Herein, a blend of the breast enlargement promoter below was used.
Hydroxyapatite (fired) of Manufacture Example 1: 5% by mass
Glycerin: 6% by mass
Polyethylene glycol: 14% by mass
Water: 75% by mass

The blend of the breast enlargement promoter described above was applied to the breast for total 9 days in continuation by 1000 mg twice per day, morning and evening. After completion of the application in the morning of Day 4, it could be felt actually that the tension of breast epidermis and the resilience of the breast improved in comparison to those before the application.

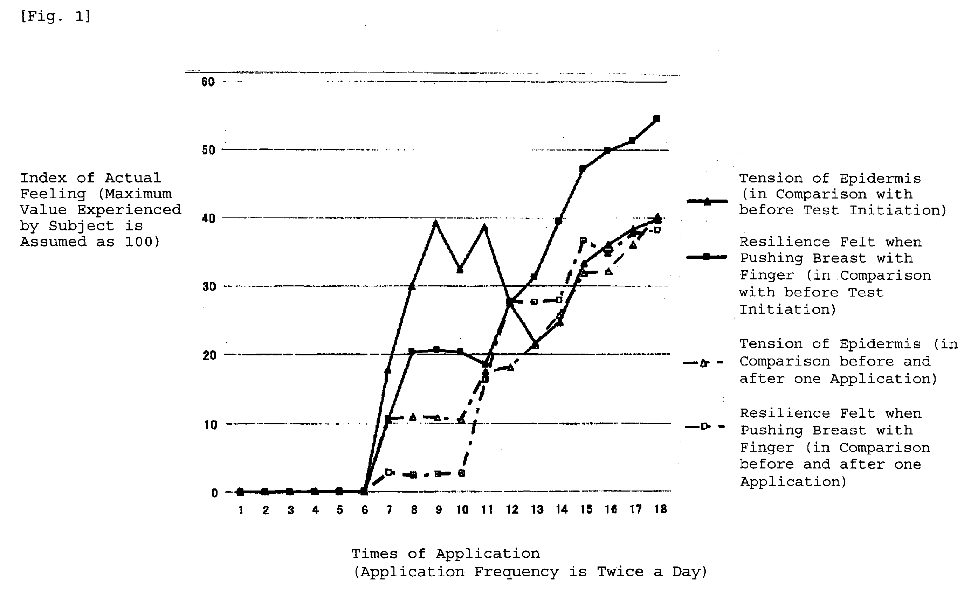

Fig. 1 shows a graph of the relationship between the number of times of the application and the index of actual feeling of the subject in the test for breast enlargement promotion described above. As the evaluation items, the "tension of the epidermis" and the "resilience felt when pushing the breast with a finger" were adopted. The "tension of the epidermis" means the tension of the skin that the subject feels sensibly, and is intended to evaluate the sign of the effects of promoting breast enlargement in the epidermis. The "resilience felt when pushing the breast with a finger" means the resilience that the subject feels when pushing the application site with a finger, and is intended to evaluate the effects on the entire skin including not only the epidermis but also the dermis.

The "index of actual feeling" adopted in the vertical axis of the graph is a digitalized value of the "tension of the epidermis" or the "resilience felt when pushing the breast with a finger" at each time of the test that is evaluated in comparison with the maximum value when the maximum value of the "tension of the epidermis" or the "resilience felt when pushing the breast with a finger" that can be experienced in the real life based on the experience of the subject, is assumed as 100.

In the evaluations, the "index of actual feeling" was obtained before and after performing each time of the application. In the plot of "in comparison to before test initiation" in the graph, a value is represented that is obtained by subtracting the index value before one application from the index value of the actual feeling after each time of the application. In the plot of the "comparison before and after one application", a value is represented that is obtained by subtracting the actual feeling index before the application of the times from the actual feeling index value after one application.

As illustrated in the graph shown in Fig. 1, actual feeling was manifested for the "tension of the epidermis" and "the resilience felt when pushing the breast with a finger" from the 7th application (the morning of Day 4). With respect to the tension of the epidermis, the tension of the epidermis continuously increased by 9th application in comparison to before the test initiation. Furthermore, from the 9th application or later, effects of improving the tension of the epidermis also lasted, which could be actually felt before and after one application. The index of the actual feeling for the resilience felt when pushing the breast with a finger continuously increased from the 7th application or later. Namely, with respect to the resilience, the resilience increased continuously, from which it is considered that collagen promoting action in the dermis is obtained intermittently and it is understood that this contributes to the effects of promoting breast enlargement.

### <Test 2 for breast enlargement promotion (effects of long term administration)>

For the purpose of investigating the effects of long term administration, the breast enlargement promoter used in Test 1 for breast enlargement promotion was administered in continuation twice, morning and evening per day for 2 months to two subjects A and B (application amount: about 500 mg per one side, specifically, about 1 g per time), and the impression after the application was recorded with respect to the questions shown below. The impressions with respect to the questions were evaluated in 9 stages in total where "no change" is stage 0, the degree of the increase is of 4 stages of +1 to +4, and the degree of the decrease is of 4 stages of -1 to -4. Details and details of the evaluations are shown in Tables 2 and 3, respectively.

[Table 2]

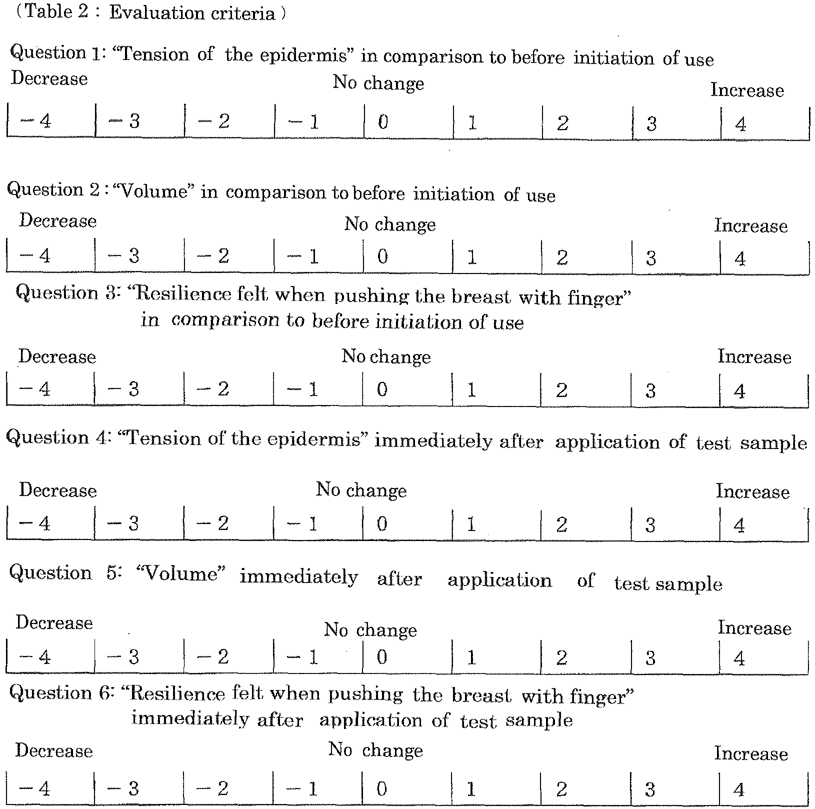

**[Table 3] Table 3**

| Question No. | Intention of Question | Subject A | Subject B |
|---|---|---|---|
| Question 1 | Question with respect to continuation property of tension | 1.8 | 2.5 |
| Question 2 | Question with respect to continuation property of volume up | 2.4 | 2.1 |
| Question 3 | Question with respect to continuation property of resilience | 2.3 | 2.3 |
| Question 4 | Question with respect to immediate effectivity of tension | 1.5 | 1.8 |
| Question 5 | Question with respect to immediate effectivity of volume up | 1.6 | 1.4 |
| Question 6 | Question with respect to immediate effectivity of resilience | 2.1 | 2.6 |

### (Discussion)

From the test continued for 2 months, there were only opinions that both of the continuation property and the immediate effectivity improved in comparison with before the use in any of the questions. With respect to the tension, the score of the immediate effectivity was equal to the score of the continuation property, suggesting that the tension obtained initially was maintained. With respect to the volume, the score relating to the continuation property was higher in comparison with the immediate effectivity, suggesting that the effects of the volume up could be obtained as the trial period passed. For any of the subjects, effects relating to improvement of the resilience (along with the immediate effectivity and the continuation property) were recognized.

### <Echo cross-sectional photography following breast enlargement test>

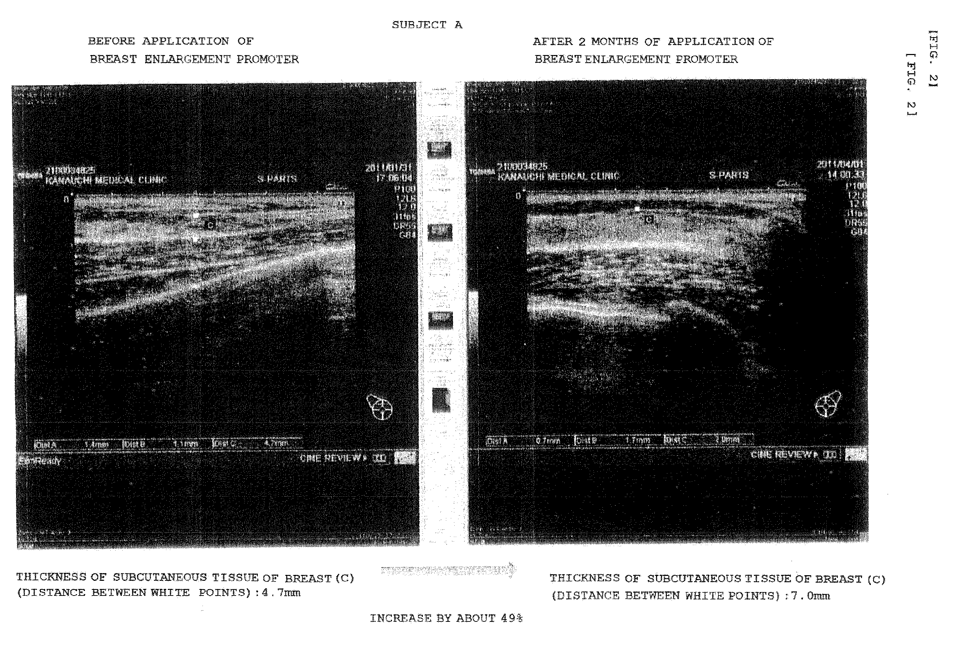

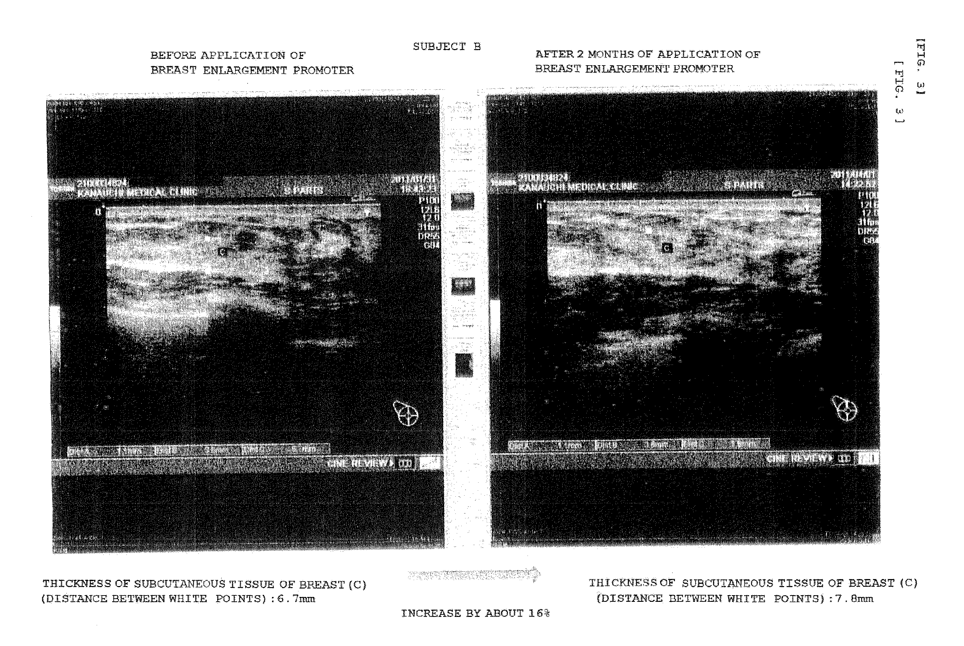

For the subj ects A and B who went through Test 2 for breast enlargement promotion, the echo cross-sectional photography was performed in order to verify the change of the breast state before and after the application. Figs. 2 and 3 show the results. The left side toward the photograph is the image taken before the application, and the (C) "distance between white points" is the thickness of the subcutaneous tissue of the breast. In contrary, the right side is the image taken after 2 months (the adaptation to the material and the interview period), and the (C) "distance between white points" is the thickness of the subcutaneous tissue of the breast. The effects that the subcutaneous tissue of the breast becomes thicker were recognized for both of the subjects.

## Claims

1. A breast enlargement promoter comprising a fine particle of calcium phosphate.

2. The breast enlargement promoter according to claim 1, wherein the fine particle of calcium phosphate is a fine particle of hydroxyapatite.

3. The breast enlargement promoter according to claim 1 or 2, wherein an average particle size of the fine particle of calcium phosphate is 10 to 1,000 nm.

4. The breast enlargement promoter according to any one of claims 1 to 3, wherein the fine particle of calcium phosphate is a sintered body.

5. The breast enlargement promoter according to claim 4, wherein the sintered body is manufactured by a method including
a mixing step in which a primary particle containing calcium phosphate is mixed with a fusion inhibitor, and
a sintering step in which the mixed particle obtained by the mixing step defined above is exposed to a sintering temperature.

6. The breast enlargement promoter according to any one of claims 1 to 5, which is for external use.
